# EUROPEAN PATENT SPECIFICATION

(11) **EP 3 731 768 B1**
(45) Date of publication and mention of the grant of the patent: **16.08.2023**
(21) Application number: 18896744.2
(22) Date of filing: 02.12.2018
(51) Int. Cl.: A61B 17/80, A61B 17/68

(54) **FRACTURE PLATE USED IN TRANSVERSE AND SEGMENTAL PATELLA FRACTURES**
BRUCHPLATTE ZUR VERWENDUNG BEI TRANSVERSALEN UND SEGMENTALEN PATELLAFRAKTUREN
PLAQUE DE FRACTURE UTILISÉE DANS DES FRACTURES TRANSVERSALES ET SEGMENTAIRES DE LA ROTULE

(30) Priority: 29.12.2017 TR 201723089
(43) Date of publication of application: 04.11.2020
(73) Proprietor: Dokuz Eylül Üniversitesi Rektörlügü, Alsancak, Izmir (TR)
(72) Inventor: KARAKASLI, Ahmet, Izmir (TR)
(86) International application number: PCT/TR2018/050755
(87) International publication number: WO 2019/132828

(56) References cited:
- WO-A1-2016/173292
- CN-A- 102 961 177
- CN-A- 106 901 821
- CN-B- 102 319 110
- CN-U- 203 677 239
- CN-U- 205 814 407
- CN-Y- 2 275 854
- CN-Y- 201 350 104
- DE-U1-202007 017 159
- US-A1- 2006 200 127
- US-A1- 2010 160 970
- US-A1- 2015 119 944

## Description

### The Related Art

Invention relates to a fracture plate used for fixation of transverse and segmental fractures, both fixing and forming compression for fracture fragments.

### Background of the Invention

Occurrence rate of patella fractures is 1 % among all fractures. Patella fracture fragments may cause 2 mm and more sliding on joint face and arthrosis and front knee pains. Gap between fracture fragments more than 3 mm may cause bleeding delays and malunion. Knee extender mechanism of patient with Patella fracture fails. Patella fractures should be anatomic reduction in order to prevent such issues and restore normal motion width of knee motions again. After providing anatomic reduction, rigid fixation should be applied and reduction consistency should be maintained. Regarding patella fractures, patella fractures are fixed by use of various fixing materials, various techniques However, the methods used at present have some disadvantages.

The method used the most commonly is tension band. In this method two Kirschner (K) wires pass through patella fracture fragment and fix the fracture fragments. K wires extends out 5 - 8 mm from bone at proximal and distal. Cerclage wire forms tension band in 8 ways by taking K wires tips inside. Cerclage wires tips are twisted and tension band is formed. In the method, both cerclage wires knot and K wires extensions may cause soft tissue and subcutaneous irritation and front knee pain. In case of giving motion earlier, loosening in cerclage wires, gap in fracture terminal, non-union may develop.

Patella fracture fragments are fixed by cannula screw and tension band is formed by knitted wire. In this method, after tightening wire, it is locked by domino and dominos may cause subcutaneous soft tissue irritation. Wires may cut bone and cause loosening. Front knee pains may develop. Since cannulated screws are longitudinally grooved required compression can be limited on patella fracture terminals.

In mesh plate fixation, particularly segmental patella fracture fixation is provided. However, compression does not develop. Because of plate wide surface, matching external surface slope of patella is difficult. Spaces between patella surface and plate cause front knee pains.

In jaw plate fixation, patella fracture fragments are fixed by plate jaw terminals from proximal and distal. In the middle section, plate proximal and distal sections are interlocked by screw. Locking screws may cause subcutaneous irritation. Jaw may not provide adequate compression on plate fracture terminals.

In the literature search, a utility model numbered CN204049810 (U) in People's Republic of China is seen. Said document discloses that splint is locked onto patella fracture and screwed by means of connection clamps. Said screws are screwed from screw holes located on splint upper surface. But screws are applied to patella onto splint upper surface. In such case, it causes stability problem. Fracture fixation becomes difficult.

During another literature search, a utility model numbered CN201888840 (U) from Peoples Republic of China is seen and it discloses a fixing component used for fixing patella fracture. It consists of multiple number of screws and screw holes. Peripherals of Patellas are fixed by a circle. Thereafter, fixer is screwed to circle and tightened. Having multiple numbers of screws damage bone tissue.

Another literature search indicated a utility model numbered CN2654047 (Y) from People's Republic of China discloses a patella fracture integration plate made from titanium alloy. The invention disclosed under said document consists of 6 integration tabs to fix patella from sides. However, not using screw for fixing reduces stability. A further fracture plate used for the fixation and compression of transverse and segmental bone fragments of a patella is known from the CN102319110B.

As a result a new fracture plate which surpasses the State-of-the-Art, overcomes the disadvantages is needed.

### Brief Description of the Invention

The invention is a new fracture plate surpassing the state of art, eliminating the disadvantages and additionally containing extra advantages.

Purpose of the invention is to disclose a new fracture plate which can be used for both segmental and transverse fractures.

Invention relates to fracture plate used for discovery of transverse and segmental fractures, both fixing and forming compression for fracture fragments.

Another purpose of the invention is to disclose a new fracture plate reducing soft tissue irritation and capable to provide compression on fracture terminals.

A further purpose of the invention is to disclose a new fracture plate minimizing pains arising from treatment and fracture post treatment of patella fractures.

Another purpose of the invention is to disclose a new fracture plate for patella fracture fixing, minimizing healing period.

A further purpose of the invention is to disclose a new fracture plate adaptable with patella and having high fixing stability for use in fixation of patella fractures.

A further purpose of the invention is to disclose a new fracture plate maintaining consistency of reduction on fracture bone terminals as well as preventing formation of gap on fracture terminals.

In order to achieve the purposes mentioned above and to be described below in the detailed description, the present invention is a fracture plate used in bone transverse and segmental patella fractures both fixing fracture fragments and forming compression. Accordingly, it is characterized in that it consists of compression screw passing through compression screw hole located on body proximal terminal and fixed onto locked plate hole located on body distal terminal, a screw driver cap providing fixation of grooved screw end into locked plate hole at said compression screw proximal terminal by tightening compression screw, grooved screw end at compression screw distal terminal providing fixation of compression screw into plate fixing hole by tightening from screw driver cap, locked plate hole located on body distal terminal fixing compression screw from grooved screw cap to distal terminal, providing tension on body when tightened from screwdriver cap, compression screw hole located on body proximal terminal and through which compression screw passes.

It is an alternative embodiment of the invention and it is characterized in that it consists of side flap providing use f plate in segmental patella fractures on body side surface, flap bone holder located on said side flap terminal section, having dent form grasping patella bone, , flap screw hole located on side flap terminal end and through which flap screw passes, flap screw passing through flap screw hole and applied to segmental fracture bones and enhancing stability.

It is a different embodiment of the invention and it is characterized in that it consists of bone holders of dent form passing into patella bone on proximal and distal ends of body and providing stability.

It is a different embodiment of invention and it is characterized in that it consists of a body having cavity fitting patella anterior joint external surface and fully sitting on patella surface and thus preventing development of irritation on skin.

It is a different embodiment of invention and it is characterized in that it consists of a compression screw which is free of groove from proximal end to distal end, only containing groove screw end at distal terminal and thus creating lag screw effect and not damaging patella bone.

### Description of Figures:

The invention will be understood better with reference to the figures attached hereto and thus the characteristics of the invention will be understood clearly. However, it does not intend to restrict the invention to these certain embodiments. In contrary, it aims to cover all alternatives, amendments and equivalences that can be included in the area which is explained by the claims attached hereto. The shown details are only for description of the preferred embodiments of the present invention and it should be understood that they are shown to describe the methods and ensuring the most useful and easy definition of the rules and conceptual characteristics of the invention. In the drawings;
- Figure 1: Is perspective view of fracture plate embodiment used in transverse patella fractures.
- Figure 2: Is a view showing flap application of fracture plate embodiment used in transverse patella fractures to patella.
- Figure 3: Is perspective view of fracture plate embodiment used in segmental patella fractures.
- Figure 4: Is view illustrating mounting of fracture plate embodiment used in segmental patella fractures.
- Figure 5: Is a view showing flap application of fracture plate embodiment used in segmental patella fractures to patella.
- Figure 6: Is a view showing flap application of fracture plate embodiment used in segmental patella fractures to patella.

The figures to understand the invention better are numbered as indicated in the attached picture and are listed below with their descriptions.

### Description of References:

**1.** Fracture plate
**10.** Body
   **11.** Bone holder
   **12.** Compression screw
      **121.** Grooved screw end
      **122.** Screwdriver cap
   **13.** Compression screw hole
   **14.** Lock plate hole
   **15.** Side flap
   **16.** Flap screw hole
   **17.** Flap bone holder
   **18.** Flap screw
**20.** Patella
   **21.** Transverse fracture
   **22.** Segmental fracture

### Detailed Description of the Invention

In this detailed description, fracture plate (1) being subject of this invention has been disclosed solely for the purpose of better understanding of the subject and with samples described in a manner not causing any restrictive effect. Scale in drawings is not exact size. They are only for better understanding of the subject. Invention relates to fracture plate (1) used for fixation of transverse and segmental patella (20) fractures, both fixing and forming compression for fracture fragments.

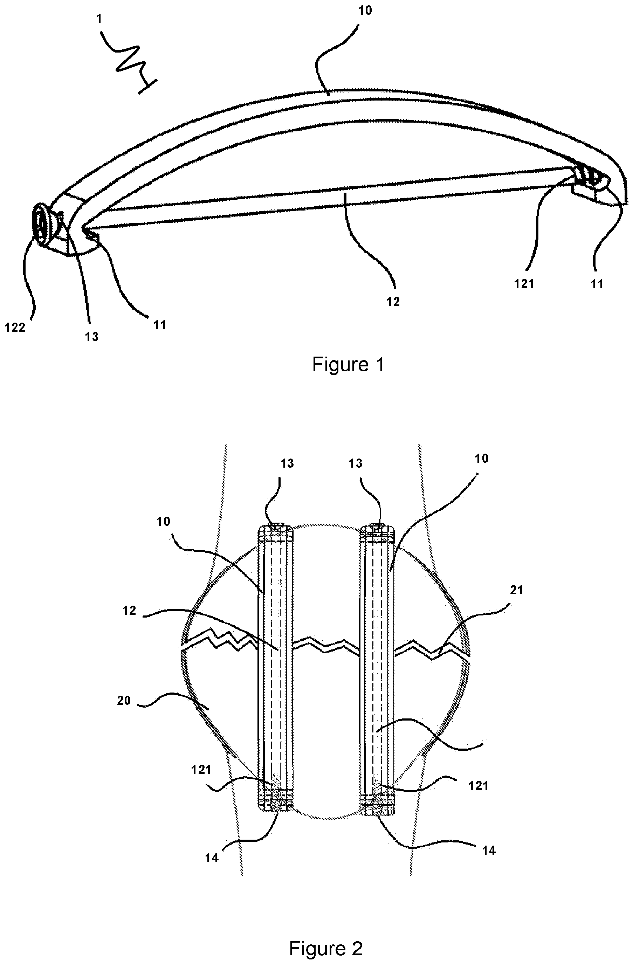

Figure 1 shows a perspective view illustrating application of fracture plate (1) embodiment used in transverse patella (20) fractures to patella. According to the figure, it consists of a body (10) having an oval form matching fracture plate (1) patella (20) shape. Body (10) has one bone holder (11) on each of both short side ends. Body (10) short edge side face has compression screw hole (13). A compression screw (12) is passed through compression screw hole (13) and fixed to lock plate hole (14) located on short edge surface on opposite side from grooved screw end (121) by a screw driver cap (122) with tightening by use of a screw driver. Lock plate hole (14) is seen clearly in figure 2.

Figure 2 shows fixation of fracture plate (1) to a patella (20) consisting of a transverse fracture (21). Two fracture plates (1) are fixed to patella (20) in the figure. It can be of more or less numbers subject to size of fracture. Figure shows a transverse fracture (21) on the patella (20) in horizontal direction. In this case, fracture plate (1) is located to patella (20) in vertical position in order to enhance stability. Fracture plate (1) inserted to patella (20) from bone holders (11) located on both ends, compression screw inserted from compression screw hole (13) located on end (proximal end) near patient's body are passed from patella (20) by tightening from screwdriver cap (122) and fixed to lock plate hole (14) located on distal end far from body by means of grooved screw end (121). When screwdriver cap (122) is tightened, tightness of fracture plate (1) increases and in this case, the transverse fracture (21) gap decreases and fracture stability increases.

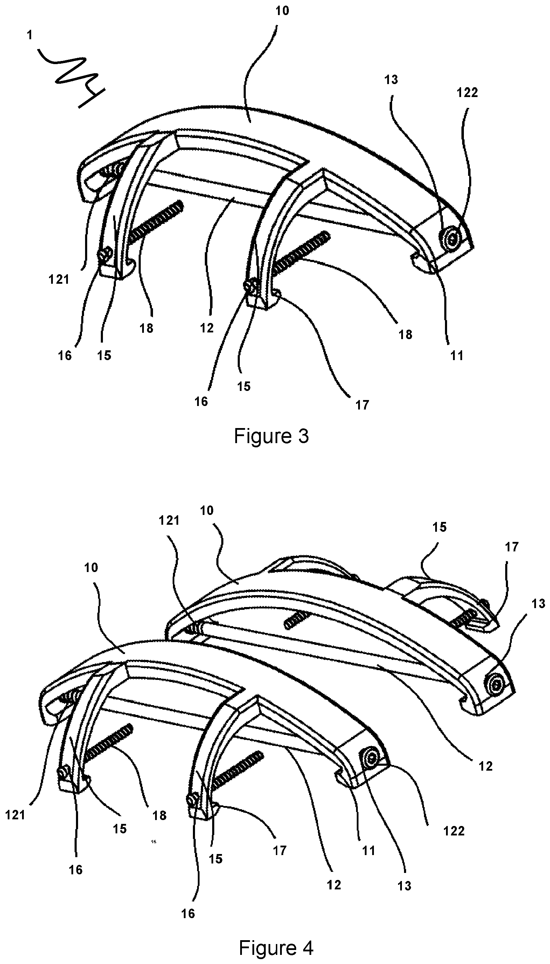

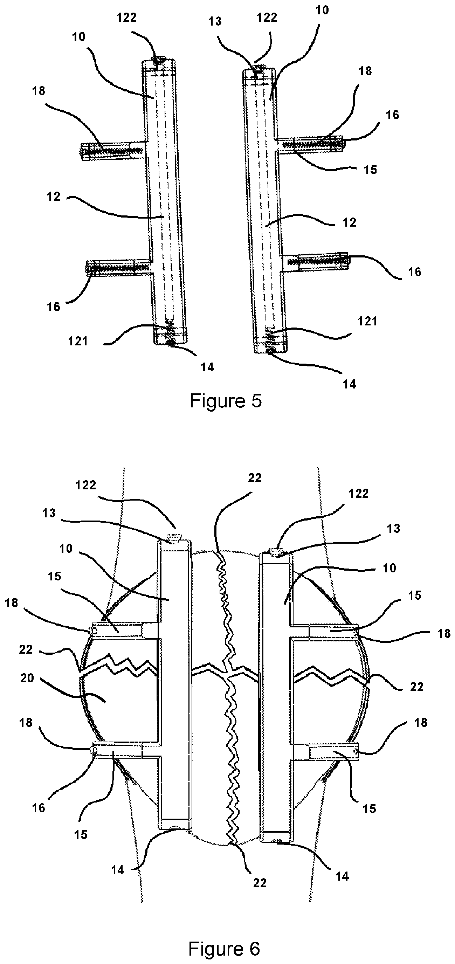

Figure 3 shows perspective view of embodiment wherein fracture plate (1) is used for segmental patella (20) fractures (22). So in order to use fracture plate (1) in segmental fractures (22), it consists of two flaps (15) on side surface of a long edge. It may consist of more or less number of side flaps (15) subject to fracture type and shape. Side flap (15) terminal section has flap bone holders (17) of clamp shapes. Back of bone holder (17) located on terminal part of side flap (15) has flap screw hole (16). Flap screw (18) is located into flap screw hole (16). Figure 4 shows perspective view of two fracture plates (1) ready for application to segmental fracture plate (22). Figure 5 shows perspective view of two fracture plates (1) ready for application to segmental fracture plate (22).

Figure 6 illustrates fixation of fracture plate (1) to segmental patella fracture (22). According to the figure, fracture plate (1) is fixed onto patella (20) bone peripherals by means of bone holders (11) located on proximal and distal terminals and flap bone holders (17) located on side flap (15) terminals. Next, compression screw (12) in compression screw hole (13) located at proximal terminal is tightened from screwdriver cap (122) and grooved screw end (121) is inserted into lock plate hole (14). Then flap screw (18) passing through flap screw hole (16) located on side flap (15) terminal is passed into segmental fracture (22) bones and fracture stability is enhanced.

The invention is a compression plate (1) developed for patella (20) fracture fixation. Patella (20) makes compression and fixation at fracture terminals and thus provides suboptimal stability. The plate maintains consistency of reduction at fracture bone terminals as well as prevents occurrence of gap at fracture ends. Thus it provides osteoinduction and speeds up fracture healing. Since this developed patella compression plate does not have embodiments that can irritate subcutaneous soft tissues, such as metal extension on external surface, screw cap extension, cerclage wire knot, no inconvenience on soft tissues and front knee pains occur. In addition, oval form of body (10) is considerably compatible with patella (20). For that reason, subcutaneous irritation does not occur. When compression screw (12) in the middle of plate (1) is tightened, compression on bone fracture ends increases. Plate (1) tightens and fixation stability increases.

## Claims

1. A fracture plate (1) used for the fixation and compression of transverse and segmental bone fragments of a patella (20), wherein the fracture plate (1) comprises:
• a body (10) having an oval form for matching the shape of the patella (20), the body comprising a compression screw hole (13) located on the proximal terminal of the body (10) and a lock plate hole (14) located on the distal end of the body (10);
• a compression screw (12) comprising a screw head (122) and a grooved screw end (121), wherein the compression screw (12) is configured to pass through the compression screw hole (13), through the bone fragments of the patella (20) and to fix to the lock plate hole (14), wherein the screw head (122) is configured to provide fixation of the grooved screw end (121) to lock to the lock plate hole (14) by means of tightening the screw head (122) at the proximal terminal of the compression screw (12) thus providing tension on the body (10), and wherein the grooved screw end (121) at the distal terminal of the compression screw (12) is configured to provide fixation of the compression screw (12) into the lock plate hole (14) by means of tightening the screw head (122).

2. The fracture plate (1) of claim 1, **characterized in that** it consists of,
• a side flap (15) providing use of plate (1) in segmental patella fractures (22) on body (10) side surface,
• a flap bone holder (17) located on said side flap (15) terminal section, having dent form grasping patella (20) bone,
• a flap screw hole (16) through which a flap screw (18) located at side flap (15) terminal section passes,
• flap screw (18) passing through flap screw hole (16) and applied to segmental fracture (22) bones and enhancing stability.

3. The fracture plate (1) of claim 1, **characterized in that** it consists of bone holders (11) of dent form passable into patella (20) bone on proximal and distal ends of body (10) and providing stability.

4. The fracture plate (1) of claim 1, **characterized in that** it consists of a body (10) having cavity fitting patella (20) anterior joint external surface and fully sitting on patella (20) surface and thus preventing development of irritation on skin.

5. The fracture plate (1) of claim 1, **characterized in that** it consists of a compression screw (12) which is free of groove from proximal end to distal end, only containing groove screw end (121) at distal terminal and thus creating lag screw effect and not damaging patella (20) bone.

## Patentansprüche

1. Frakturplatte (1), die zur Fixierung und Kompression von transversalen und segmentalen Knochenfragmenten einer Patella (20) verwendet wird, wobei die Frakturplatte (1) umfasst:
• einen Körper (10) mit einer ovalen Form zur Anpassung an die Form der Patella (20), wobei der Körper ein Kompressionsschraubenloch (13), das sich am proximalen Ende des Körpers (10) befindet, und ein Verriegelungsplattenloch (14), das sich am distalen Ende des Körpers (10) befindet, umfasst;
• eine Kompressionsschraube (12) mit einem Schraubenkopf (122) und einem gerillten Schraubenende (121), wobei die Kompressionsschraube (12) so konfiguriert ist, dass sie durch das Kompressionsschraubenloch (13) und durch die Knochenfragmente der Patella (20) hindurchgeht und am Verriegelungsplattenloch (14) befestigt wird, wobei der Schraubenkopf (122) so konfiguriert ist, dass er eine Fixierung des gerillten Schraubenendes (121) zur Verriegelung am Verriegelungsplattenloch (14) durch Anziehen des Schraubenkopfes (122) am proximalen Ende der Kompressionsschraube (12) ermöglicht, wodurch eine Spannung auf den Körper (10) ausgeübt wird, und wobei das gerillte Schraubenende (121) am distalen Ende der Kompressionsschraube (12) so konfiguriert ist, dass es die Fixierung der Kompressionsschraube (12) in dem Verriegelungsplattenloch (14) durch Anziehen des Schraubenkopfes (122) ermöglicht.

2. Frakturplatte (1) nach Anspruch 1,
**dadurch gekennzeichnet, dass** sie aus
• einer Seitenklappe (15), die die Verwendung der Platte (1) bei segmentalen Patellafrakturen (22) auf der Seitenfläche des Körpers (10) ermöglicht,
• einem Klappen-Knochenhalter (17), der an dem Endabschnitt der Seitenklappe (15) angeordnet ist und eine gezahnte Form aufweist, die den Patella-Knochen (20) erfasst,
• einem Klappenschraubenloch (16), durch das eine am Endabschnitt der Seitenklappe (15) befindliche Klappenschraube (18) verläuft,
• einer Klappenschraube (18) besteht, die durch das Klappenschraubenloch (16) hindurchgeht und an einer segmentalen Fraktur (22) der Knochen angebracht ist und die Stabilität erhöht.

3. Frakturplatte (1) nach Anspruch 1, **dadurch gekennzeichnet, dass** sie aus Knochenhaltern (11) in gezahnter Form besteht, die in den Patellaknochen (20) am proximalen und distalen Ende des Körpers (10) eingeführt werden können und für Stabilität sorgen.

4. Frakturplatte (1) nach Anspruch 1, **dadurch gekennzeichnet, dass** sie aus einem Körper (10) besteht, der einen Hohlraum aufweist, der an die Außenfläche des vorderen Gelenks der Patella (20) angepasst ist und vollständig auf der Oberfläche der Patella (20) aufliegt, wodurch die Entstehung von Hautreizungen verhindert wird.

5. Frakturplatte (1) nach Anspruch 1, **dadurch gekennzeichnet, dass** sie aus einer Kompressionsschraube (12) besteht, die vom proximalen Ende bis zum distalen Ende keine Rille aufweist und nur am distalen Ende ein Rillenschraubenende (121) enthält, wodurch ein Zugschraubeneffekt entsteht und der Patellaknochen (20) nicht beschädigt wird.

## Revendications

1. Plaque de fracture (1) utilisée pour la fixation et la compression de fragments osseux transversaux et segmentaires d'une rotule (20), dans lequel la plaque de fracture (1) comprend :
• un corps (10) ayant une forme ovale pour faire correspondre la forme de la rotule (20), le corps comprenant un trou de vis de compression (13) situé sur l'extrémité proximale du corps (10) et un trou de plaque de verrouillage (14) situé sur l'extrémité distale du corps (10) ;
• une vis de compression (12) comprenant une tête de vis (122) et une extrémité de vis rainurée (121), dans lequel la vis de compression (12) est configurée pour passer à travers le trou (13) de la vis de compression, à travers les fragments osseux de la rotule (20) et pour se fixer au trou de la plaque de verrouillage (14), dans lequel la tête de vis (122) est configurée pour assurer la fixation de l'extrémité rainurée de la vis (121) au trou de la plaque de verrouillage (14) en serrant la tête de vis (122) à l'extrémité proximale de la vis de compression (12), fournissant ainsi une tension sur le corps (10), et dans lequel l'extrémité rainurée de la vis (121) à la borne distale de la vis de compression (12) est configurée pour assurer la fixation de la vis de compression (12) dans le trou de la plaque de verrouillage (14) au moyen du serrage de la tête de la vis (122).

2. La plaque de fracture (1) de la revendication 1,
**caractérisée en ce qu'**elle consiste en
• un volet latéral (15) fournissant l'utilisation de la plaque (1) dans les fractures segmentaires de la rotule (22) sur la surface latérale du corps (10),
• un porteur (17) d'os de volet situé sur ladite section terminale de volet latéral (15), ayant une forme de dent qui saisit l'os de la rotule (20),
• un trou de vis de volet (16) par lequel passe une vis de volet (18) située dans la section terminale du volet latéral (15),
• vis de volet (18) passant par le trou de vis de volet (16) et appliquée aux os de la fracture segmentaire (22) et renforçant la stabilité.

3. La plaque de fracture (1) de la revendication 1, **caractérisée en ce qu'**elle est constituée de supports osseux (11) de forme dentée passant dans l'os de la rotule (20) aux extrémités proximale et distale du corps (10) et assurant la stabilité.

4. La plaque de fracture (1) de la revendication 1, **caractérisée en ce qu'**elle consiste en un corps (10) ayant une cavité s'adaptant à la surface externe de l'articulation antérieure de la rotule (20) et reposant entièrement sur la surface de la rotule (20) et empêchant ainsi le développement d'une irritation sur la peau.

5. La plaque de fracture (1) de la revendication 1, **caractérisée en ce qu'**elle consiste en une vis de compression (12) dépourvue de rainure de l'extrémité proximale à l'extrémité distale, ne contenant qu'une extrémité de vis à rainure (121) à l'extrémité distale, et créant ainsi un effet de vis tire-fonds et n'endommageant pas l'os de la rotule (20).
